# EUROPEAN PATENT APPLICATION

(11) **EP 1 683 538 A1**
(43) Date of publication of application: **26.07.2006**
(21) Application number: 05100450.5
(22) Date of filing: 25.01.2005
(51) Int. Cl.: A61M 5/315, A61M 5/24

(54) **Injection evice for administering a medication liquid**

(71) Applicant: Pfizer Health AB, 112 87 Stockholm (SE)
(72) Inventor: The designation of the inventor has not yet been filed
(74) Representative: Courgeon, Antoine

(57) **Abstract**

The invention relates to an injection device for administering multiple doses of a medication liquid, said device (1) comprising a housing (2), a cartridge (6) containing a preparation or precursor component for the preparation and having a front part and a rear part, the rear part (10) being closed by a movable wall (12), a displacement of which wall causes the preparation to be moved forward the other end of the cartridge (6), through which the liquid exits, and actuating means (16) for actuating the wall (12) whose forward movement inside the housing is driven by an actuator (16) at the proximal end (18) of the housing (2) and adapted to be actuated by the user, said actuator (16) being able to move between a pulled out first position corresponding to arming of the injection device (1) and a pushed in second position corresponding to injecting a dose of liquid medication, said injection device (1) being characterised in that the actuator (16) is able to assume a third position, different from the first and second positions, informing the user that the injection device (1) is ready to deliver the final dose of liquid medication.

## Description

The present invention relates to an injection device for administering multiple doses of a medication liquid, which device is adapted to alert the user to the fact that he is preparing to administer to another person or to himself the final dose of the medication liquid contained in the device.

### Background of the invention

Injection devices of the above kind are known in the art. They enable a user to inject multiple doses of a medication liquid from an cartridge containing the liquid. To this end, the user may adjust the volume of doses to be injected subsequently as a function of medical prescriptions. As a general rule, the volume of the final dose contained in the cartridge will be less than the prescribed volume. It is therefore necessary to find an effective way to warn the user that he is preparing to administer to another person or to himself the final dose of medication liquid and, where applicable, that the volume of that final dose does not correspond to the prescribed volume that he has programmed.

### Summary of the Invention

An object of the present invention is to respond to this requirement by providing an injection device for administering multiple doses of a medication liquid, said device comprising a housing, a cartridge containing a preparation or precursor component for the preparation and having a front part and a rear part, the rear part being closed by a movable wall, a displacement of which wall causes the preparation to be moved forward the other end of the cartridge, through which the liquid exits, and actuating means for actuating the wall whose forward movement inside the housing is driven by an actuator at the proximal end of the housing and adapted to be actuated by the user, said actuator being able to move between a pulled out first position corresponding to arming of the injection device and a pushed in second position corresponding to injecting a dose of liquid medication, said injection device being characterised in that the actuator is able to assume a third position, different from the first and second positions, informing the user that the injection device is ready to deliver the final dose of liquid medication.

Thanks to these features, the present invention provides an injection device that indicates clearly and intelligibly to the user that he is preparing to deliver the final dose of medication liquid. This indication to the user takes the form of an unusual event, namely the actuator adopting a position different from its usual pulled out and pushed in positions. This event immediately alerts the user, who realises that he is about to administer to another person or to himself the final dose of medication liquid.

According to a complementary feature of the invention, the actuator automatically assumes its third position at the moment when the user pulls out the actuator to arm the injection device for the final time.

The event informing the user that he is dispensing the final dose of liquid therefore occurs automatically, in the context of normal use of the injection device. The user therefore has no need to concern himself with how full the cartridge is, and is warned in good time that said cartridge contains only one dose of the liquid.

### Brief description of the drawings

Other features and advantages of the present invention will emerge more clearly from the following detailed description of one embodiment of an injection device of the invention, given by way of purely illustrative and non-limiting example and with reference to the appended drawings, in which:
- figure 1 is a side view of the injection device of the invention, showing in section the cartridge containing the liquid to be administered with the actuator in its pulled out position corresponding to the ordinary dispensing of a dose of medication liquid;
- figure 2 is a view analogous to that of figure 1, showing the actuator in an unusual pulled out position corresponding to dispensing the final dose;
- figure 3 is a front view of the injection device of the invention;
- figure 4 is a view in section taken along the line C-C in figure 3 of the injection device of the invention;
- figure 5 is a view in section taken along the line D-D in figure 3 of the injection device of the invention; and
- figures 6 and 7 are views to a larger scale of the ringed areas Y and Z in figure 5.

### Detailed description of the preferred embodiments

The present invention stems from the general inventive concept of associating the dispensing of the final dose of a medication liquid by means of an injection device with an event that is unusual in the context of normal operation of the device, in order to alert the user. In this way, the user is warned that he is preparing to administer to another person or to himself the final dose of medication, and may take care to check that the volume of the final dose corresponds to the prescribed volume. If it does not, he must administer a complementary injection using a new injection device. The injection device of the invention is therefore very safe to use and prevents the remaining medication liquid being wasted.

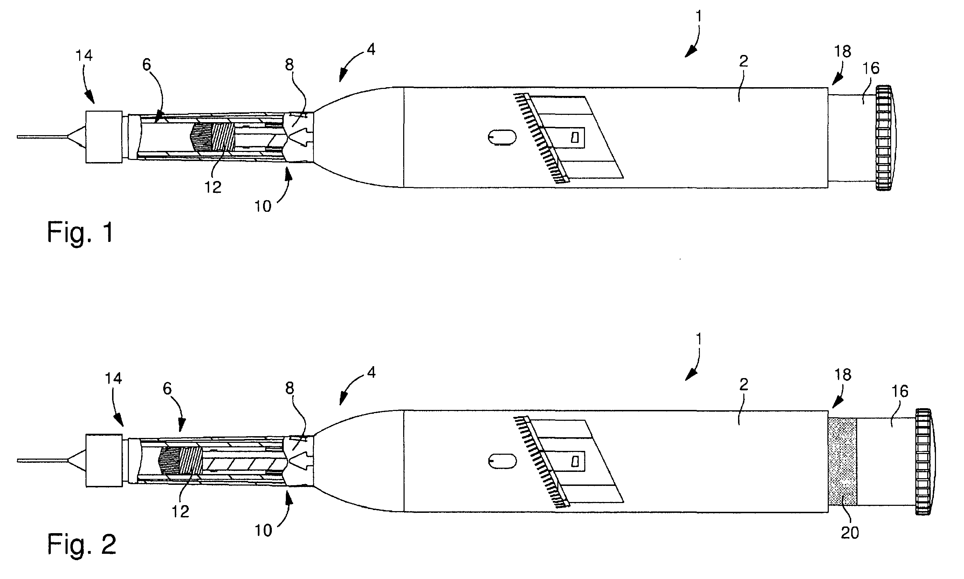

Figure 1 is a profile view of the injection device 1 of the invention, which comprises a generally elongate body 2) preferably adapted to be held by a user. To a distal end 4 of the body 2 is fixed an cartridge 6 protected by a plastic cap 8 and containing the medication liquid to be administered. The cartridge has one end 10 closed by a sealed wall 12 that is adapted to be moved toward the other end 14 of the cartridge 6, through which the liquid exits. The wall 12 is moved by an actuator 16 at a proximal end 18 of the body 2 and adapted to be operated by the user.

In the position represented in figure 1, the injection device 1 is ready to dispense a dose of liquid medication. To this end, the user has had to pull out the actuator 16 until it stops moving in a position in which it is partly withdrawn from the body 2 of said injection device 1.

In the position shown in figure 2, the injection device 1 is ready to dispense the final dose of medication liquid. This situation differs from that shown in figure 1 in that the actuator 16 occupies a second pulled out position different from the first pulled out position corresponding to dispensing an ordinary dose. The actuator 16 protrudes farther from the body 2 of the injection device 1 than in the situation represented in figure 1. This unusual event alerts the user and informs him that the dose he is preparing to administer to another person or to himself is the final one. The user must then measure the volume of the remaining dose. If that volume corresponds to the prescribed volume, no particular problem arises. Otherwise, the user must administer a complementary injection using a second injection device 1. The unusual nature of the greater protrusion of the actuator 16 before dispensing the final dose of medication liquid may be strengthened by providing on the body of the actuator 16 a ring 20 a different colour from said actuator 16, for example.

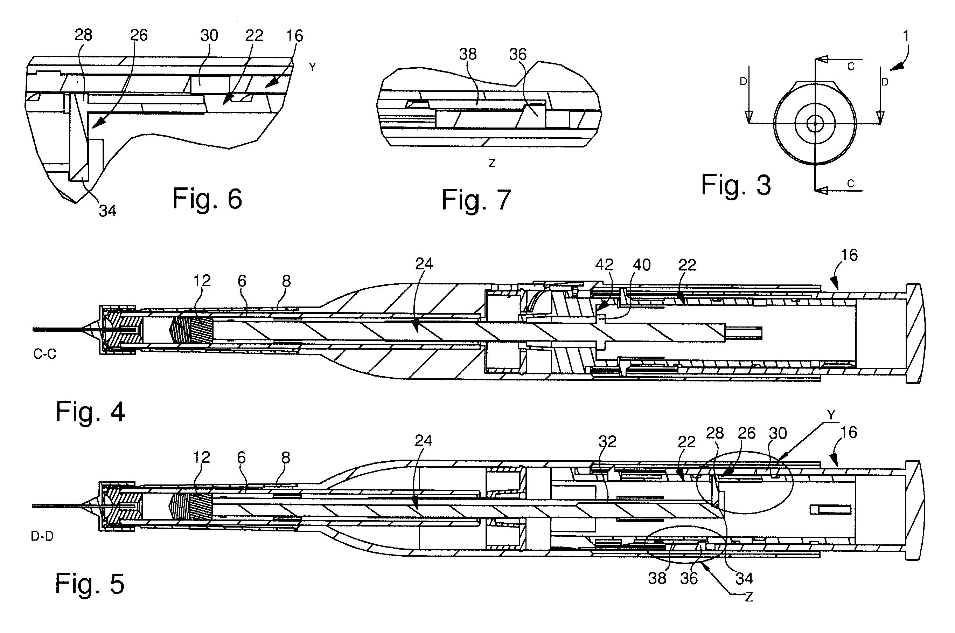

Referring to figure 4, it is seen that forward movement imparted to the actuator 16 by the user is transmitted to the wall 12 via a bush 22 and a plunger 24. When preparing a dose, the user pulls out the actuator 16 until it stops moving. During this movement, the bush 22, which is attached to said actuator 16, also moves backwards. On the other hand, the plunger 24 does not move. When administering a dose of medication liquid, the user applies pressure to the actuator 16, which is depressed inside the body 2, entraining the bush 22 in its forward movement. In turn, the bush 22 causes forward movement of the plunger 24 which in turn pushes the wall 12 forward to expel a dose of liquid from the cartridge 6.

The bush 22 comprises a locking member in the form of a ratchet 26 whose head 28 projects into a housing 30 in the body of the actuator 16 so that during ordinary use of the injection device 1 of the invention the bush 22 and the actuator 16 are fastened together. The ratchet 26 also co-operates with a cam profile 32 on the plunger 24 ending in a cavity 34. Before dispensing the final dose of medication liquid, the user pulls the actuator 16 out again. At this moment, the relative position of the bush 22 and the plunger 24 is such that the ratchet 26 that was tracking the cam profile 32 drops into the cavity 34 and is disengaged from the housing 30, thereby releasing the actuator 16, which may continue to move backwards. This backward movement of the actuator 16 is interrupted when an abutment 36 on the inside face of said actuator 16 reaches the end of a groove 38 in the facing face of the bush 22.

Accordingly, at the moment of preparing the final dose of the medication liquid to be administered, the actuator 16 protrudes farther from the body 2 of the injection device 1 than in ordinary use of said device 1, which alerts the user to the fact that he is preparing to administer to another person or to himself the final dose of liquid. This occurs automatically, without the user having at any time to concern himself with the volume of liquid remaining in the cartridge 6. If the final dose corresponds to the prescribed volume, there is no particular problem. Otherwise, the user must administer a complementary injection using a new injection device.

When the abutment 36 reaches the end of the groove 38, the actuator 16 and the bush 22 are again constrained to move together in the axial direction and only the ratchet 26 accommodated in the cavity 34 in the plunger 24 opposes complete extraction of these two components, which is insufficient. There is therefore provided on said plunger 24 a flange 40 against which an annular shoulder 42 on the bush 22 abuts, which prevents demounting the injection device 1 of the invention.

It goes without saying that the present invention is not limited to the embodiments that have just been described, and that the person skilled in the art may envisage various simple modifications and variations that lie within the scope of the invention as defined by the appended claims.

## Claims

1. An injection device for administering multiple doses of a medication liquid, said device (1) comprising a housing (2), a cartridge (6) containing a preparation or precursor component for the preparation and having a front part and a rear part, the rear part (10) being closed by a movable wall (12), a displacement of which wall causes the preparation to be moved forward the other end of the cartridge (6), through which the liquid exits, and actuating means (16) for actuating the wall (12) whose forward movement inside the housing is driven by an actuator (16) at the proximal end (18) of the housing (2) and adapted to be actuated by the user, said actuator (16) being able to move between a pulled out first position corresponding to arming of the injection device (1) and a pushed in second position corresponding to injecting a dose of liquid medication, said injection device (1) being **characterised in that** the actuator (16) is able to assume a third position, different from the first and second positions, informing the user that the injection device (1) is ready to deliver the final dose of liquid medication.

2. An injection device according to claim 1, **characterised in that** the actuator (16) automatically assumes its third position at the moment when the user pulls out the actuator (16) to arm the injection device (1) for the final time.

3. An injection device according to either claim 1 or claim 2, **characterised in that**, when the actuator (16) is in its third position, it uncovers a ring (20) that is a different colour from said actuator (16).

4. An injection device according to any of claims 1 to 3, **characterised in that** forward movement imparted to the actuator (16) by the user is transmitted to the wall (12) via a bush (22) and a plunger (24).

5. An injection device according to claim 4, **characterised in that**, during ordinary use of said injection device (1), the actuator (16) and the bush (22) are fastened together and, at the moment the user pulls the actuator (16) out a final time before dispensing the final dose of medication liquid, said actuator (16) is released and is therefore able to continue its backward movement.

6. An injection device according to claim 5, **characterised in that** the bush (22) comprises a locking member for attaching the actuator (16) to said bush (22) during ordinary use of the injection device (1).

7. An injection device according to claim 6, **characterised in that** the locking member is a ratchet (26) having a head (28) projecting into a housing (30) in the actuator (16), said ratchet also co-operating with a cam profile (32) on the plunger (24) and ending in a cavity (34), the relative position of the bush (22) and the plunger (24) at the moment when the user arms the injection device (1) for the final time being such that the ratchet (26) drops into the cavity (34) and is disengaged from the housing (30), thereby releasing the actuator (16), which is able to continue its backward movement.

8. An injection device according to claim 7, **characterised in that** the backward movement of the actuator (16) is interrupted when an abutment (36) on said actuator (16) reaches the end of a groove (38) in the bush (22).

9. An injection device according to claim 8, **characterised in that**, at the moment when the abutment (36) reaches the end of the groove (38), an annular shoulder (42) on the bush (22) abuts against a flange (40) on the plunger (24).
